(19)

Europäisches Patentamt

European Patent Office

Office européen des brevets

(11) **EP 4 177 278 A1**

(12) **EUROPEAN PATENT APPLICATION**
published in accordance with Art. 153(4) EPC

(43) Date of publication:
**10.05.2023 Bulletin 2023/19**

(21) Application number: **21833888.7**

(22) Date of filing: **02.07.2021**

(51) International Patent Classification (IPC):
**C08F 14/18** (2006.01)     **C08F 2/01** (2006.01)
**G01N 33/44** (2006.01)

(52) Cooperative Patent Classification (CPC):
**C08F 2/01; C08F 14/18; G01N 33/44**

(86) International application number:
**PCT/JP2021/025156**

(87) International publication number:
**WO 2022/004880 (06.01.2022 Gazette 2022/01)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **03.07.2020 JP 2020115514**

(71) Applicant: **Daikin Industries, Ltd.**
**Osaka-shi, Osaka 530-8323 (JP)**

(72) Inventors:
• **TACHIKAWA, Shouhei**
**Osaka-shi, Osaka 530-8323 (JP)**

• **YAMADA, Takaya**
**Osaka-shi, Osaka 530-8323 (JP)**
• **SHIGENAI, Fumiko**
**Osaka-shi, Osaka 530-8323 (JP)**
• **KAWABE, Rumi**
**Osaka-shi, Osaka 530-8323 (JP)**
• **MORITA, Shingo**
**Osaka-shi, Osaka 530-8323 (JP)**

(74) Representative: **Hoffmann Eitle**
**Patent- und Rechtsanwälte PartmbB**
**Arabellastraße 30**
**81925 München (DE)**

(54) **PREDICTION DEVICE, CALCULATION DEVICE, MANUFACTURING DEVICE, AND MANUFACTURING METHOD**

(57)     A variation in the performance value of a polymer being manufactured can be reduced. A prediction device(10A) that, in manufacturing of a polymer, predicts a performance value indicating performance of the polymer in a polymerization tank after a raw material is fed, and may include: an acquisition unit(111) that acquires, as a prediction observation value, an observation value observed, in current manufacturing of the polymer, as a value related to the manufacturing of the polymer; and a prediction unit(112) that predicts the performance value of the polymer being currently manufactured at a predetermined timing, from the prediction observation value acquired by the acquisition unit, by using a relation between an observation value acquired in past manufacturing of the same type of polymer as the polymer, and a performance value of the polymer at the predetermined timing in the past manufacturing.

FIG.2

## Description

Technical Field

The present disclosure relates to a prediction device, a calculation device, a manufacturing device, and a manufacturing method that are used in manufacturing of a polymer.

Background Art

Conventionally, soft sensors are used in various fields. A soft sensor estimates a quantity by using a process variable that is relatively easy to measure, such as temperature or pressure, in place of a process variable $y1$ that is difficult to measure and is not frequently measured, or a process variable $y2$ that needs time to be measured. For example, Patent Literature 1 discloses a system for pelletizing polyolefin resin, including a soft sensor that estimates a melt flow rate.

Citation List

Patent Literature

Patent Literature 1: JP 2011-245742 A

Summary

Technical Problem

The present disclosure provides a prediction device, a calculation device, a manufacturing device, and a manufacturing method that can reduce the number of steps required in a manufacturing process for acquiring a polymer with a specific performance value.

Solution to Problem

A prediction device of the present disclosure may be a prediction device that, in manufacturing of a polymer, predicts a performance value indicating performance of the polymer in a polymerization tank after a raw material is fed, and may include: an acquisition unit that acquires, as a prediction observation value, an observation value observed, in current manufacturing of the polymer, as a value related to the manufacturing of the polymer; and a prediction unit that predicts the performance value of the polymer being currently manufactured at a predetermined timing, from the prediction observation value acquired by the acquisition unit, by using a relation between an observation value acquired in past manufacturing of the same type of polymer as the polymer, and a performance value of the polymer at the predetermined timing in the past manufacturing.

In the prediction device, the prediction unit may predict the performance value of the polymer being currently manufactured at the predetermined timing, from the prediction observation value acquired by the acquisition unit, by using a trained model that has learned, through machine learning, a relation between a training observation value acquired in past manufacturing of the same type of polymer as the polymer, and a performance value of the polymer at the predetermined timing in the past manufacturing.

In the prediction device, the acquisition unit may acquire the observation value along with elapsed time since start of the current manufacturing, and uses a set of the elapsed time and the observation value for the prediction observation value, and the prediction unit may predict the performance value of the polymer being currently manufactured at the predetermined timing, by using a relation between a training observation value that is a set of the observation value in the past manufacturing and elapsed time since start of the past manufacturing, and the performance value at the predetermined timing in the past manufacturing.

In the prediction device, the acquisition unit may predict the performance value of the polymer at the predetermined timing, by using the trained model that is trained by using at least one observation value or performance value suitable for prediction of the performance value, the at least one observation value or performance value being selected, based on a genetic algorithm, from a plurality of types of observation values acquired in the past manufacturing and performance values of the polymer acquired in the past manufacturing.

In the prediction device, the observation value may include at least any of a stirring electric current value of a stirrer that stirs a reaction mixture including the raw material in the polymerization tank, a stirring electric power value, a pressure value in the polymerization tank, an amount of any of raw materials fed from start of manufacturing until a current time point, and an amount of all raw materials fed from start of manufacturing until a current time point.

In the prediction device, the performance value may be any of a Mooney viscosity, an average molecular weight, a specific gravity, a hardness, an elongation, a minimum torque, a maximum torque, an induction time to start of curing, an optimal curing time, a tensile strength, a 100% tensile stress, a glass-transition temperature, an initial pyrolysis temperature, and a compression set rate.

In the prediction device, the polymer may be an elastomer.

In the prediction device, the predetermined timing may be a timing at which a fed amount of a predetermined raw material of the polymer being manufactured becomes a predetermined value, or a timing at which a predetermined time period passes after start of manufacturing.

A calculation device of the present disclosure may be a calculation device that, in manufacturing of a polymer, obtains a target timing at which a specific operation is performed for terminating the manufacturing of the polymer in a polymerization tank, and may include: a receiving unit that receives a target performance value indicating a target of performance of the polymer in current manufacturing, and a performance value at a predetermined timing after a raw material of the polymer is fed and before the manufacturing is terminated; and a calculation unit that obtains the target timing at which the polymer being currently manufactured achieves the target performance value, by using a relation among a performance value of the polymer at the predetermined timing in past manufacturing of the same type of polymer as the polymer, a performance value of the polymer acquired in the past manufacturing, and a target timing in the past manufacturing, and by using the target performance value and the performance value at the predetermined timing received by the receiving unit.

In the calculation device, the calculation unit may obtain the target timing at which the polymer being currently manufactured achieves the target performance value, by using a second trained model that has learned, through machine learning, the relation among the performance value of the polymer at the predetermined timing in the past manufacturing of the same type of polymer as the polymer, the performance value of the polymer acquired in the past manufacturing, and the target timing in the past manufacturing, and by using the target performance value and the performance value at the predetermined timing.

In the calculation device, the calculation device may be connected to the prediction device as described above, and the receiving unit may receive, as the performance value at the predetermined timing, the performance value predicted by the prediction device.

Another calculation device of the present disclosure may be a calculation device that, in manufacturing of a polymer, obtains a target timing used to determine termination of the manufacturing of the polymer, and may include: a receiving unit that receives, as a calculation observation value, an observation value observed, in current manufacturing, as a value related to the manufacturing of the polymer, and receives, as a target value, a target performance value indicating a target of performance of the polymer in the current manufacturing; and a calculation unit that obtains a target timing in the current manufacturing, from the calculation observation value received by the receiving unit, by using a relation among an observation value acquired in past manufacturing of the same type of polymer as the polymer, a performance value of the polymer acquired in the past manufacturing, and a target timing in the past manufacturing.

In the calculation device, the calculation unit may obtain the target timing, from the calculation observation value, by using a third trained model that has learned, through machine learning, a relation among a training observation value acquired in past manufacturing of the same type of polymer as the polymer, a performance value of the polymer acquired in the past manufacturing, and a target timing in the past manufacturing.

In the calculation device, the polymer may be an elastomer.

A manufacturing device of the present disclosure may include: a polymerization tank into which a raw material of a polymer is fed; an acquisition unit that acquires, as a prediction observation value, an observation value observed, in current manufacturing of the polymer, as a value related to the manufacturing of the polymer; a prediction unit that predicts a performance value of the polymer being currently manufactured at a predetermined timing, from the prediction observation value acquired by the acquisition unit, by using a relation between an observation value acquired in past manufacturing of the same type of polymer as the polymer, and a performance value of the polymer at the predetermined timing in the past manufacturing; a receiving unit that receives a target performance value indicating a target of performance of the polymer in the current manufacturing, and the performance value at the predetermined timing predicted by the prediction unit; a calculation unit that obtains a target timing at which the polymer being currently manufactured achieves the target performance value, by using a relation among the performance value of the polymer at the predetermined timing in the past manufacturing of the same type of polymer as the polymer, a performance value of the polymer acquired in the past manufacturing, and a target timing in the past manufacturing, and by using the target performance value and the performance value at the predetermined timing received by the receiving unit; and a control unit that terminates the current manufacturing, by using the target timing obtained by the calculation unit.

A manufacturing method of the present disclosure may include: feeding a raw material of a polymer into a polymerization tank; acquiring, as a prediction observation value, an observation value observed, in current manufacturing of the polymer, as a value related to the manufacturing of the polymer; predicting a performance value of the polymer being currently manufactured at a predetermined timing, from the acquired prediction observation value, by using a relation between an observation value acquired in past manufacturing of the same type of polymer as the polymer, and a performance value of the polymer at the predetermined timing in the past manufacturing; receiving a target performance value indicating a target of performance of the polymer in the current manufacturing, and the predicted performance value at the predetermined timing; obtaining a target timing at which the polymer being currently manufactured achieves the target performance value, by using a relation among the performance value of the polymer at the predetermined timing in the past manufacturing of the same type of polymer as the polymer, a performance value of the polymer acquired in the past manufacturing, and a target timing in the past manufacturing, and by using the received target performance value and the received performance value at the predetermined timing; and terminating the current manufacturing, by using the obtained target timing.

Generalized specific embodiments of the foregoing may be implemented by any one of a system, a method, and a computer program, or a combination thereof.

Advantageous Effect of Invention

According to the prediction device, the calculation device, the manufacturing device, and the manufacturing method of the present disclosure, the number of steps required in the process of manufacturing a polymer can be reduced.

Brief Description of Drawings

[Figure 1] Figure 1 is a schematic diagram showing a configuration of a manufacturing device in an embodiment 1.
[Figure 2] Figure 2 is a block diagram showing a configuration of a prediction device in the embodiment 1.
[Figure 3] Figure 3 is a block diagram showing a configuration of a calculation device in the embodiment 1.
[Figure 4] Figure 4 is a flowchart for describing processing in the manufacturing device in the embodiment 1.
[Figure 5A] Figure 5A is a schematic diagram for describing learning by a Mooney viscosity prediction model used by the prediction device in the embodiment 1.
[Figure 5B] Figure 5B is a schematic diagram for describing input to and output from the Mooney viscosity prediction model used by the prediction device in the embodiment 1.
[Figure 6] Figure 6 is a schematic diagram for describing teaching data for the Mooney viscosity prediction model used by the prediction device in the embodiment 1.
[Figure 7A] Figure 7A is a schematic diagram for describing learning by a target timing calculation model used by the prediction device in the embodiment 1.
[Figure 7B] Figure 7B is a schematic diagram for describing input to and output from the target timing calculation model used by the prediction device in the embodiment 1.
[Figure 8] Figure 8 is a schematic diagram showing a configuration of a manufacturing device in an embodiment 2.
[Figure 9] Figure 9 is a block diagram showing a configuration of a calculation device in the embodiment 2.
[Figure 10] Figure 10 is a flowchart for describing processing in the manufacturing device in the embodiment 2.

[Figure 11A] Figure 11A is a schematic diagram for describing learning by a Mooney viscosity prediction model used by a prediction device in the embodiment 2.
[Figure 11B] Figure 11B is a schematic diagram for describing input to and output from the Mooney viscosity prediction model used by the prediction device in the embodiment 2.

Description of Embodiments

An example of a method for manufacturing a polymer is that raw materials of a polymer to be manufactured are fed into a polymerization tank, and the polymer is acquired by polymerization reaction in the polymerization tank. In such cases, feeding of the raw materials into the polymerization tank is performed not only by feeding all raw materials at a time, but a raw material is fed halfway through manufacturing in some cases. For example, among the raw materials, which include a monomer and an initiator, some can be fed initially, some can be fed intermittently, some can be fed continuously, and so forth. There are also some cases where different methods are used in combination respectively for a plurality of types of raw materials that are used to generate a polymer. Specifically, when a polymer is manufactured by using a raw material A and a raw material B, the raw material A may be fed only initially, while the raw material B may be fed intermittently.

Such manufacturing of a polymer is not always finished after the same manufacturing time period, and requires 50 hours on one occasion, requires 70 yours on another occasion, and so forth even if the same polymer is manufactured, so that each process step in manufacturing cannot be determined only depending on time. For example, a manufacturing process advances to a next step when the fed amount of a specific raw material reaches a predetermined amount, in some cases. Moreover, for example, the process advances to a next step on condition that the performance of a polymer in the course of manufacturing reaches a predetermined value, in some cases. For example, the case where the process advances to a next step depending on the fed amount of a raw material can be relatively easily implemented by observing the fed amount of the raw material. On the other hand, when the process advances to a next step depending on a predetermined value related to the performance of a polymer in the course of manufacturing, it is difficult to observe in real time the performance value of the polymer in the course of manufacturing. Accordingly, it is difficult to track each situation in manufacturing of a polymer, so that the polymer being manufactured tends to vary in performance.

To observe the performance value of a polymer being manufactured, it is necessary to sample, from a polymerization tank, and perform measurement for the polymer being manufactured. However, an operation of sampling the polymer from the polymerization tank requires no small amount of operation time.

Incidentally, in manufacturing of a polymer, many types of materials are used, and many types of equipment are used. Moreover, a long time is required for manufacturing. Accordingly, there are many candidates for data that can be used by a soft sensor. It is therefore indeterminate which data is preferred when used by the soft sensor, so that it is also difficult to easily use the soft sensor.

A prediction device, a calculation device, a manufacturing device, and a manufacturing method according to each embodiment, which will be described below with reference to the drawings, reduce the number of steps required in a manufacturing process. The "prediction device" of the present disclosure predicts, in manufacturing of a polymer, a "performance value" that specifies performance of the polymer at a predetermined timing, by using an observation value acquired as a value related to the manufacturing. By using the prediction device, a performance value can be predicted in manufacturing of a polymer, without actually sampling, from a polymerization tank, and measuring the polymer. Accordingly, for example, a degree of progress of polymerization in manufacturing of a polymer can be found, without actually measuring the polymer.

The "calculation device" obtains, in manufacturing of a polymer, a "target timing" that is used to determine termination of the manufacturing of the polymer, by using a performance value of the polymer at a predetermined timing, or an observation value acquired in the manufacturing. By using the calculation device, a target timing can be determined in manufacturing of a polymer, without actually sampling the polymer from a polymerization tank and measuring performance of the polymer.

The "manufacturing method" and the "manufacturing device" manufacture a polymer by using a predicted performance value and an obtained target timing. In a description below, the same components are denoted by the same signs, and a description thereof is omitted.

A "polymer" being manufactured by the manufacturing device is a polymer that is a compound obtained as a result of polymerization of one or more types of monomers. In the following description, the "polymer" is assumed to be, but is not limited to, an "elastomer". For example, the "elastomer" is a "fluoroelastomer", a "non-fluoroelastomer", or the like.

In one embodiment, the polymer is a fluoroelastomer, and more particularly a perfluoroelastomer.

The glass-transition temperature of the fluoroelastomer may be 25°C or less, preferably 0°C or less, more preferably -5°C or less, even more preferably -10°C or less, or may be -20°C or less. Here, the glass-transition temperature is acquired as follows. A DSC curve is acquired by using a differential scanning calorimeter (X-DSC 823e, manufactured by Hitachi Technoscience Corporation), by lowering the temperature to -75°C and then raising the temperature at 20°C/minute, and a temperature corresponding to a point where an extended line from a base line of the DSC curve around a second-order transition intersects with a tangent line to the DSC curve at an inflection point is used for the glass-transition temperature.

The fluoroelastomer may be, for example, an elastomer including a structural unit derived from at least one monomer selected from the group consisting of tetrafluoroethylene (TFE), vinylidene fluoride (VdF), and perfluoroethylene unsaturated compounds (for example, hexafluoropropylene (HFP), perfluoro(alkyl vinyl ether) (PAVE), and the like) represented by a following formula (1):

$$CF_2 = CF-R_f^a \qquad (1)$$

where $R_f^a$ is -CF$_3$ or -OR$_f^b$, where $R_f^b$ is a perfluoroalkyl group with one to five carbons.

The fluoroelastomer may be perfluoroelastomer including tetrafluoroethylene (TFE) such as tetrafluoroethylene (TFE)/perfluoroalkyl vinyl ether fluoroelastomer, vinylidene fluoride (VdF) fluoroelastomer, TFE/propylene (Pr) fluoroelastomer, TFE/Pr/VdF fluoroelastomer, ethylene (Et)/hexafluoropropylene (HFP) fluoroelastomer, Et/HFP/VdF fluoroelastomer, Et/HFP/TFE fluoroelastomer, fluorosilicone fluoroelastomer, fluorophosphazene fluoroelastomer, or the like.

The perfluoroelastomer may be formed of TFE/PAVE or the like. The composition of TFE/PAVE is preferably (50 to 90)/(50 to 10) (mol%), more preferably (50 to 80)/(50 to 20) (mol%), even more preferably (55 to 75)/(45 to 25) (mol%).

PAVE in such a case may be, for example, perfluoromethyl vinyl ether (PMVE), perfluoropropyl vinyl ether (PPVE), or the like, which can be used in isolation, or in arbitrary combination.

The percentage of fluorine in the fluoroelastomer is preferably 50 mass% or more, more preferably 55 mass% or more, even more preferably 60 mass% or more. The upper limit of the percentage of fluorine content is not limited, and is preferably 71 mass% or less.

The above-mentioned examples of the fluoroelastomer are compositions of a main monomer, and those copolymerized with a monomer giving a crosslinkable group can also be used suitably. The monomer giving a crosslinkable group may be any monomer that can introduce an appropriate crosslinkable group, according to a manufacturing method or a crosslink system, and may be, for example, a known polymerizable compound including an iodine atom, a bromine atom, a carbon-carbon double bond, a cyano group, a carboxyl group, a hydroxyl group, an amino group, an ester group, or the like, or may be a chain transfer agent or the like.

The non-fluoroelastomer may be diene rubber, such as acrylonitrile-butadiene rubber (NBR) or hydride thereof (HNBR), styrene-butadiene rubber (SBR), chloroprene rubber (CR), butadiene rubber (BR), natural rubber (NR), or isoprene rubber (IR), or may be ethylene-propylene-termonomer copolymer rubber, silicone rubber, butyl rubber, epichlorohydrin rubber, acrylic rubber, chlorinated polyethylene (CPE), polymer alloy of acrylonitrile-butadiene rubber and vinyl chloride (PVC-NBR), ethylene-propylene-diene monomer (EPDM) rubber, or the like.

In the following description, a "performance value" is assumed to be, but is not limited to, a "Mooney viscosity" of an elastomer. Specifically, the performance value may be any value that indicates performance of a polymer. For example, the "performance value" is any of "average molecular weight", "specific gravity relative to 4°C water as a reference (hereinafter, simply referred to as "specific gravity")", "hardness", "elongation", "minimum torque", "maximum torque", "induction time to start of curing", "optimal curing time", "tensile strength", "100% tensile stress", "glass-transition temperature", "initial pyrolysis temperature", and "compression set rate" of a polymer being manufactured. The performance value may be a value obtained based on a function by using a plurality of ones selected from among the above-mentioned performance values.

A "target timing" is a timing at which a specific operation for terminating manufacturing is performed, and is assumed here to be a "timing at which a specific operation is performed to achieve a target performance value of an elastomer when manufacturing is terminated". The specific operation for terminating manufacturing is, for example, stopping feeding a raw material, stopping stirring, reducing pressure in the tank, starting cooling, or the like. The "target timing" needs to be obtained according to a situation. For example, the "target timing" can be obtained according to an observation value observed as a value related to manufacturing of a polymer. "Start" of manufacturing is determined, for example, by a first timing of feeing a raw material. "Termination" of manufacturing corresponds to a timing at which, after the target timing is reached, a manufactured polymer becomes able to be extracted or transported due to a predetermined condition for termination being met. The "condition for termination" is that, for example, polymerization substantially ceases, the inside of the polymerization tank becomes a predetermined temperature, the inside of the polymerization tank becomes a predetermined pressure value, a predetermined time period passes after last feeding, or the like.

In manufacturing of an elastomer, an "observation value" is a value observed, in manufacturing of a polymer, as a value related to the manufacturing of the polymer, and is, for example, a measurement value measured by a sensor or the like installed in the manufacturing device, or a value obtained based on a calculation formula (a result of calculation) by using such a measurement value. For example, when a temperature sensor or a pressure sensor is used, measurement values measured by the sensors are also observation values. Moreover, for example, a "total amount of a raw material fed from start until a current time point", and a "total amount of all raw materials fed from start until a current time point" are also observation values. Note that a plurality of types of observation values may be used in prediction of the performance value and calculation of the target timing, and the number of observation values used is not limited. A plurality of different types of observation values acquired at the same time may be used, and some of types of observation values listed above may be used in combination.

[Embodiment 1]

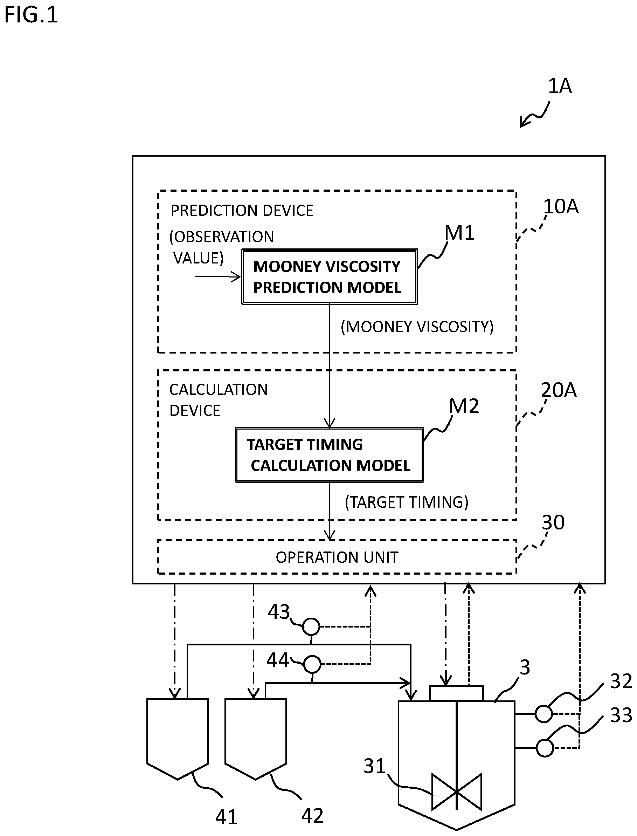

A manufacturing device 1A according to an embodiment 1 is described by using Figures 1 to 7B. Figure 1 is an example of the manufacturing device 1A used in manufacturing of an elastomer. The manufacturing device 1A shown in Figure 1 includes: a prediction device 10A that predicts, by using a soft sensor, a Mooney viscosity that is a performance value specifying performance of an elastomer in a polymerization tank 3, in manufacturing of the elastomer that is manufactured by feeing raw materials into the polymerization tank 3; and a calculation device 20A that obtains, by using a soft sensor, a target timing in the manufacturing of the elastomer in the polymerization tank 3. Moreover, the manufacturing device 1A includes an operation unit 30 that performs a specific operation for terminating the current manufacturing by using the target timing obtained by the calculation device 20A.

The raw materials fed into the manufacturing device 1A include a monomer, an initiator, a chain transfer agent, and the like. Moreover, of the raw materials, some are fed initially, some are fed intermittently, some are fed continuously, and so forth, and the manufacturing device 1A according to the embodiment 1 is described by using a raw material that is fed intermittently or a raw material that is fed continuously, as an example.

Specifically, as shown in Figure 1, the prediction device 10A predicts, from an "observation value", a "Mooney viscosity" that is a performance value of the elastomer at a predetermined timing in manufacturing, by using a Mooney viscosity prediction model M1 that is a first soft sensor. The calculation device 20A calculates a "target timing" in the manufacturing of the elastomer from the "Mooney viscosity" at the predetermined timing, by using a target timing calculation model M2 that is a second soft sensor. The manufacturing device 1A can finish feeding the material into the polymerization tank 3 by using the obtained target timing, and thus can terminate the manufacturing of the elastomer. For example, after the target timing is reached, the manufacturing device 1A can put the elastomer into an extractable state or the like, by finishing stirring or finishing adjusting temperature in the polymerization tank 3.

The manufacturing device 1A includes the polymerization tank 3 used for elastomer polymerization, and can output a control signal for controlling a stirrer 31 and receive a result of the control (for example, the number of rotations of the stirrer and the amount of electric current used for the rotations). Moreover, storage tanks 41, 42 that store the raw material are connected to the manufacturing device 1A, and the manufacturing device 1A can output a control signal for controlling feeding of the raw material. For example, the manufacturing device 1A can control a timing of feeding from the storage tanks 41, 42 to the polymerization tank 3, by using, as an observation value, a pressure value in the polymerization tank 3 measured by the pressure sensor 33.

In the following description, as an example, the manufacturing device 1A uses the Mooney viscosity prediction model M1 that is a trained model and the target timing calculation model M2 that is a trained model, for the first soft sensor and the second soft sensor, respectively, which, however, are not limited to the above-mentioned models. The first soft sensor may be a function that simply receives a required "observation value" as an input and predicts a "Mooney viscosity" of the elastomer at a predetermined timing. The second soft sensor may also be a function that simply receives the "Mooney viscosity" of the elastomer at the predetermined timing and a target "performance value" of the elastomer being manufactured as inputs and calculates a "target timing".

<Prediction device>

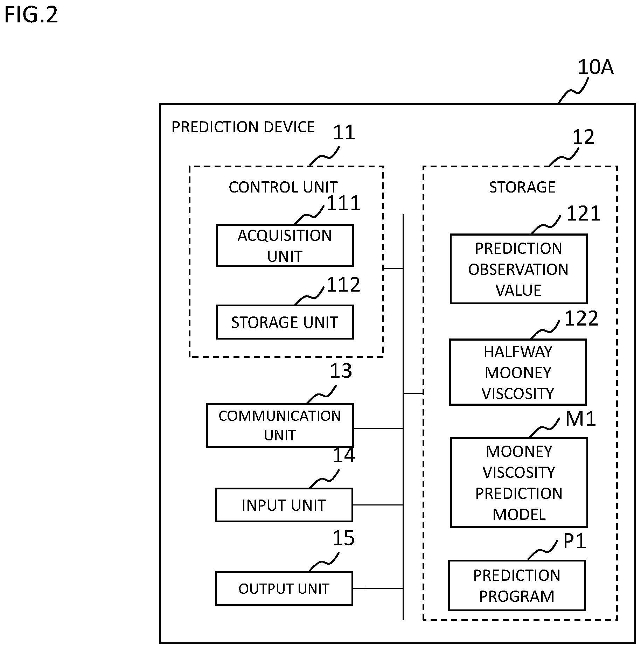

As shown in Figure 2, the prediction device 10A is an information processing device including a control unit 11, a storage 12, and a communication unit 13. The control unit 11 is a controller that controls the entire prediction device 10A. For example, the control unit 11 implements processing as an acquisition unit 111 and a prediction unit 112 by reading and executing a prediction program P1 stored in the storage 12. The control unit 11 is not limited to a unit that implements a predetermined function through cooperation between hardware and software, but may be a hardware circuit designed exclusively to implement the predetermined function. In other words, the control unit 11 can be implemented by using any of various processors such as CPU, MPU, GPU, FPGA, DSP, and ASIC.

The storage 12 is a recording medium storing various information. For example, the storage 12 is implemented by using a RAM, a ROM, a flash memory, an SSD (Solid State Drive), a hard disk, or any other storage device, or by combining any of such devices as appropriate. In addition to the prediction program P1 to be executed by the control unit 11, various data and the like to be used for learning and prediction are stored in the storage 12. For example, the storage 12 stores a prediction observation value 121, a halfway Mooney viscosity 122, the Mooney viscosity prediction model M1, and the prediction program P1. Note that the Mooney viscosity prediction model M1 may be included in the prediction program P1.

The communication unit 13 is an interface circuit (module) for enabling data communication with an external device (not shown).

The prediction device 10A can include an input unit 14 and an output unit 15. The input unit 14 is input devices, such as an operation button, a mouse, or a keyboard, used for input of an operation signal and data. The output unit 15 is output devices, such as a display, used for output of a result of processing and data, and other purposes.

The acquisition unit 111 acquires, as the prediction observation value 121, an observation value observed as a value related to manufacturing of the elastomer in current manufacturing. The acquisition unit 111 stores the prediction observation value 121 in the storage 12.

At the time, the acquisition unit 111 may acquire the observation value along with time information related to the current manufacturing, and may use a set of the time information and the observation value for the prediction observation value. The time information is, typically, elapsed time since start of the current manufacturing. Note that the time information may be other time information than the elapsed time. In other words, instead of the elapsed time, time information at which the manufacturing is started and time information at which each observation value is observed can be used for elapsed time since start of the manufacturing.

The prediction unit 112 predicts a Mooney viscosity of the elastomer being currently manufactured at a predetermined timing, from the prediction observation value acquired by the acquisition unit 111, by using a relation between an observation value acquired in past manufacturing of the same type of elastomer as the elastomer being currently manufactured and a Mooney viscosity of the elastomer at the predetermined timing in the past manufacturing. The "predetermined timing" is a timing after the raw material is fed and before the manufacturing is finished, and is, for example, a timing at which the fed amount of a predetermined raw material becomes a predetermined value, a timing at which a predetermined time period (for example, three hours) passes after start of the manufacturing, or the like. Note that in the following description, a "Mooney viscosity at the predetermined timing" is referred to as "halfway Mooney viscosity" as necessary.

The prediction unit 112 stores the predicted halfway Mooney viscosity 122 in the storage 12. Alternatively, the prediction unit 112 may output the halfway Mooney viscosity 122 to the calculation device 20A.

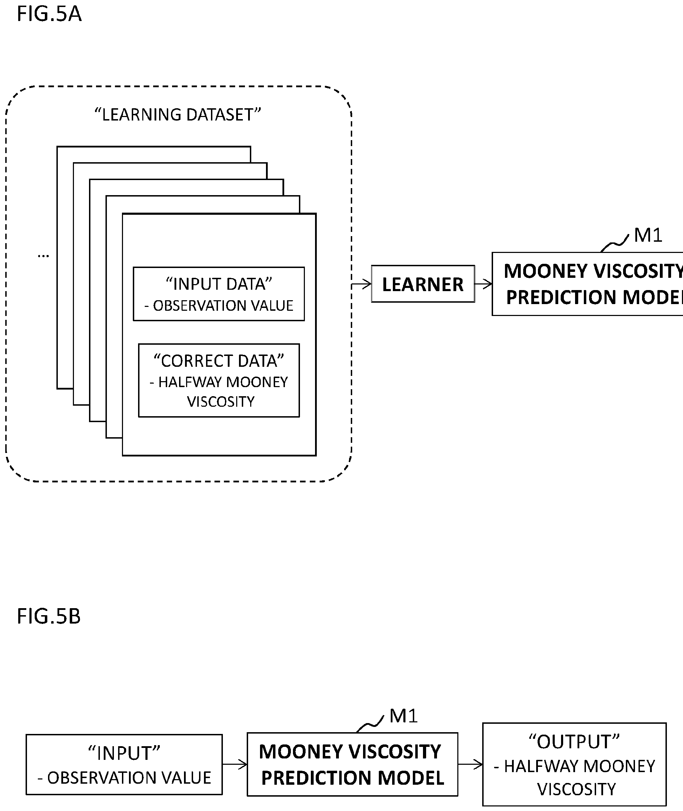

Here, the prediction unit 112 can use the Mooney viscosity prediction model M1, which is a trained model that has been trained through machine learning, in prediction of the halfway Mooney viscosity 122. The Mooney viscosity prediction model M1 has learned a relation between a training observation value, which is an observation value acquired in past manufacturing of the same type of elastomer as the elastomer being manufactured, and a halfway Mooney viscosity in the past manufacturing. Accordingly, the Mooney viscosity prediction model M1 can predict the halfway Mooney viscosity of the elastomer being manufactured, by receiving, as an input, the prediction observation value acquired by the acquisition unit 111. The machine learning will be described later by using Figure 5A.

For example, when the Mooney viscosity prediction model M1 is a model using multiple regression analysis, the prediction-target "halfway Mooney viscosity" is an "objective variable" of the regression analysis, and the "observation value" is an "explanatory variable" of the regression analysis. The prediction observation value 121 that is input data of the Mooney viscosity prediction model M1 may be one observation value, or a combination of a plurality of observation values, selected, based on a genetic algorithm, from a plurality of types of observation values acquired in past manufacturing. Accordingly, the prediction observation value 121 selected based on the genetic algorithm is an observation value suitable for prediction of a Mooney viscosity. Specifically, when the Mooney viscosity prediction model M1 is built by using a selected observation value, the observation value is selected by using the genetic algorithm such that prediction performance of the Mooney viscosity prediction model M1 becomes higher.

For example, the prediction observation value 121 includes at least any of a "stirring electric current value" of the stirrer 31 that stirs a reaction mixture including the raw material in the polymerization tank 3, a "stirring electric power value", a "pressure value" in the polymerization tank 3, a "fed amount" of any of raw materials from start of the manufacturing until a current time point, and a "fed amount" of all raw materials from start of the manufacturing until a current time point. In other words, the prediction observation value 121 may be a value of one type, or a combination of values of multiple types, selected from among a plurality of the above-mentioned types.

Note that the prediction observation value 121 may be a measurement value actually measured by any of the sensors 32, 33 and flowmeters 43, 44, or a value calculated by using the measurement value. In such a case, for example, the acquisition unit 111 obtains the prediction observation value 121 by using the acquired measurement value, based on a predetermined calculation formula, and stores the prediction observation value 121 in the storage 12.

When the prediction observation value 121 includes the elapsed time along with the observation value, the prediction unit 112 predicts the halfway Mooney viscosity of the elastomer being manufactured in the current manufacturing, by using a relation between a training observation value as a set of the observation value in the past manufacturing and the elapsed time since start of the past manufacturing, and the halfway Mooney viscosity in the past manufacturing.

The halfway Mooney viscosity 122 is thus predicted by the prediction device 10A, whereby the Mooney viscosity of the elastomer being manufactured can be found, without a complicated process, such as sampling from the polymerization tank 3 and measuring the elastomer during the manufacturing. Since termination of the manufacturing of the elastomer can be determined by using the halfway Mooney viscosity, the number of manufacturing process steps can be reduced.

<Calculation device>

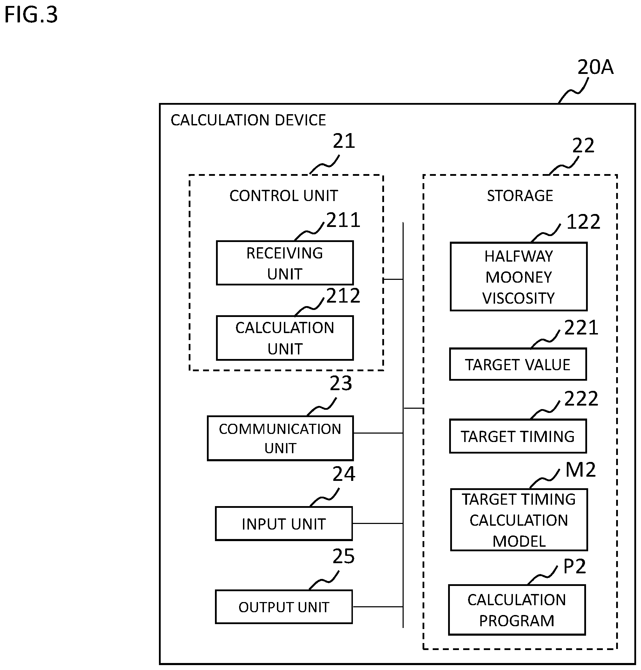

As shown in Figure 3, the calculation device 20A is an information processing device including a control unit 21, a storage 22, and a communication unit 23. Moreover, the calculation device 20A can include an input unit 24 and an output unit 25. For example, the control unit 21 implements processing as a receiving unit 211 and a calculation unit 212, by reading and executing a calculation program P2 stored in the storage 22. The storage 22 stores, for example, the halfway Mooney viscosity 122, a target value 221, a target timing 222, the target timing calculation model M2, and the calculation program P2. Note that the target timing calculation model M2 may be included in the calculation program P2.

The receiving unit 211 receives a performance value (halfway Mooney viscosity) of the elastomer at a predetermined timing, and a target Mooney viscosity of the elastomer in current manufacturing. In the following description, the "target Mooney viscosity" is referred to as "target value" as necessary. For example, the halfway Mooney viscosity 122 received by the receiving unit 211 may be the halfway Mooney viscosity predicted by the prediction device 10A. The receiving unit 211 stores the received halfway Mooney viscosity 122 and the received target value 221 in the storage 22.

For example, when an elastomer with the same performance is manufactured multiple times, the calculation device 20A does not need to receive a new target value each time, but may read and use the target value 221 received in the past and stored in the storage 22.

The calculation unit 212 obtains a target timing used to determine termination of the current manufacturing of the elastomer, by using a relation among a halfway Mooney viscosity in past manufacturing of the same type of elastomer as the elastomer being manufactured, a final Mooney viscosity of the elastomer acquired in the past manufacturing, and a target timing in the manufacturing of the elastomer, and by using the target value and the halfway Mooney viscosity received by the receiving unit 211. The calculation unit 212 outputs the obtained target timing to the operation unit 30. Note that all of the halfway Mooney viscosity, the final Mooney viscosity, and the target timing in the past manufacturing are actually measured values.

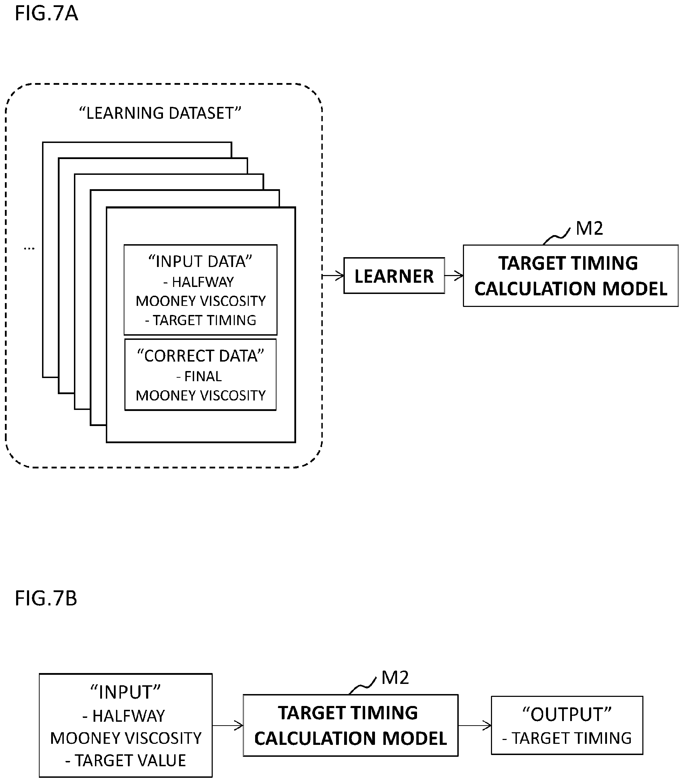

Here, the calculation unit 212 can use the target timing calculation model M2, which is a trained model that has been trained through machine learning, in calculation of the target timing 222. The target timing calculation model M2 has learned, through machine learning, a relation among a past halfway Mooney viscosity of the same type of elastomer as the elastomer being manufactured, a final Mooney viscosity, and a target timing. Accordingly, by using the target value 221 and the halfway Mooney viscosity 122 received by the receiving unit 211, the target timing calculation model M2 can obtain the target timing used to determine termination of the current manufacturing of the elastomer, in order to allow performance of the elastomer being currently manufactured to be the target value. The machine learning will be described later by using Figure 7A.

The target timing 222 is thus obtained by the calculation device 20A, whereby the manufacturing process can be terminated when the Mooney viscosity of the elastomer is in a target state. Accordingly, by using the target timing 222 obtained as a result of the calculation, variation in the Mooney viscosity of the elastomer being manufactured can be reduced, compared to a conventional simple soft sensor. Note that even if the manufacturing is terminated based on the target timing obtained by the calculation device 20A by using the target value, the Mooney viscosity (final Mooney viscosity) of the acquired elastomer does not necessarily coincide with the target value, but comes within a desired range from the target value.

The above description uses an example in which the receiving unit 211 receives the halfway Mooney viscosity predicted by the prediction device 10A. However, when the elastomer being manufactured can be sampled from the polymerization tank 3 and actually measured, the receiving unit 211 may receive an actually measured halfway Mooney viscosity. In particular, when the prediction device 10A cannot accurately predict a halfway Mooney viscosity due to impossibility of acquiring an accurate observation value as a result of an abnormality occurring in equipment or the like that acquires an observation value, it is effective to stop using the prediction device 10A, and use a halfway Mooney viscosity of the elastomer being manufactured that is sampled from the polymerization tank 3.

Although the prediction device 10A, the calculation device 20A, and the operation unit 30 are shown as separate entities in the example in Figure 1, two or more of the entities may be configured by using a single information processing device. For example, all the entities may be configured by using a single information processing device, or the prediction device 10A and the calculation device 20A, or the calculation device 20A and the operation unit 30, may be configured by using a single information processing device.

<Manufacturing method>

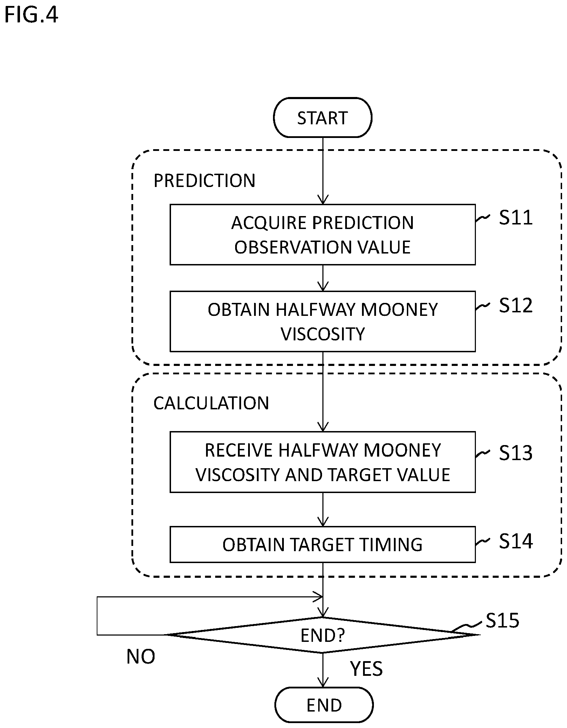

Elastomer manufacturing processing in the manufacturing device 1A is described by using a flowchart of Figure 4. When the manufacturing device 1A starts manufacturing an elastomer, the acquisition unit 111 of the prediction device 10A acquires a prediction observation value 121 (S11). Note that the acquisition unit 111 may acquire the prediction observation value 121 through calculation by using a value acquired from an external sensor or device.

The prediction unit 112 obtains a halfway Mooney viscosity 122 of the elastomer being manufactured, by using the prediction observation value 121 acquired in step S11 (S12). Thus, the manufacturing device 1A can acquire the halfway Mooney viscosity, without actual measurement through a complicated process.

The receiving unit 211 of the calculation device 20A receives the halfway Mooney viscosity 122 obtained in step S12 and a target value 221 of the elastomer being manufactured (S13).

The calculation unit 212 obtains a target timing 222 used to determine termination of the manufacturing of the elastomer being manufactured, by using the halfway Mooney viscosity 122 and the target value 221 received in step S13 (S14).

Thus, by using the target timing 222 obtained in step S14, the manufacturing device 1A continues manufacturing of the elastomer until a condition for termination is met, and terminates the manufacturing of the elastomer when the condition is met (S15).

Thus, the manufacturing device 1A can reduce the number of manufacturing process steps because a sample inspection is not needed and a step of the manually performed inspection can therefore be eliminated.

Note that the processing in steps S11 and S12 is performed by the prediction device 10A, and the processing in steps S13 and S14 is performed by the calculation device 20B. If it is possible for the manufacturing device 1A to actually measure a halfway Mooney viscosity, in step S13, the receiving unit 211 may receive a halfway Mooney viscosity actually measured by sampling the elastomer being manufactured, and in step S14, the calculation unit 212 may obtain a target timing by using the actually measured halfway Mooney viscosity. In such a case, the target timing at which the target value is achieved can be obtained from a soft sensor acquired based on a rule of past experiences, by using the sampled halfway Mooney viscosity.

Here, the manufacturing device 1A may be implemented by a single computer, or may be implemented by a combination of a plurality of computers connected through a network. For example, the prediction device 10A shown in Figure 2 and the calculation device 20A shown in Figure 3 do not need to be different computers, but may be implemented by using the same computer. In such a case, the control units 11, 21 are the same unit, as well as the storages 12, 22, the communication units 13, 23, the input units 14, 24, and the output units 15, 25. Moreover, for example, all or part of the data stored in the storage 12 may be stored in an external recording medium connected through a network, and the prediction device 10A may be configured to use the data stored in the external recording medium.

<Machine learning>

An outline of creation and use of the Mooney viscosity prediction model M1 using machine learning is illustrated by using Figures 5A and 5B, and an outline of creation and use of the target timing calculation model M2 using machine learning is illustrated by using Figures 7A and 7B. The "machine learning" is a scheme for creating a "model" that learns a feature included in input data and infers a result corresponding to new input data. Specifically, the machine learning is performed by a "learner". The thus created model is referred to as "trained model".

As shown in Figure 5A, a plurality of pairs of input data and correct data acquired in past manufacturing of the elastomer are inputted into the "learner". Specifically, when the Mooney viscosity prediction model M1 is created, an "observation value" acquired in past manufacturing of the elastomer is used for input data, and a "halfway Mooney viscosity" actually measured in the past manufacturing of the elastomer is used for correct data. Thus, the learner learns a relation between such observation values and halfway Mooney viscosities, which is a learning dataset relation, and creates the Mooney viscosity prediction model M1 that is a trained model indicating the relation between the observation values and the halfway Mooney viscosities with parameters. By using the created Mooney viscosity prediction model M1, a "halfway Mooney viscosity" that is a desired output can be obtained for an "observation value" that is a new input, as shown in Figure 5B. Accordingly, an output obtained by the Mooney viscosity prediction model M1 is a "halfway Mooney viscosity" as a prediction value, not an actually measured value.

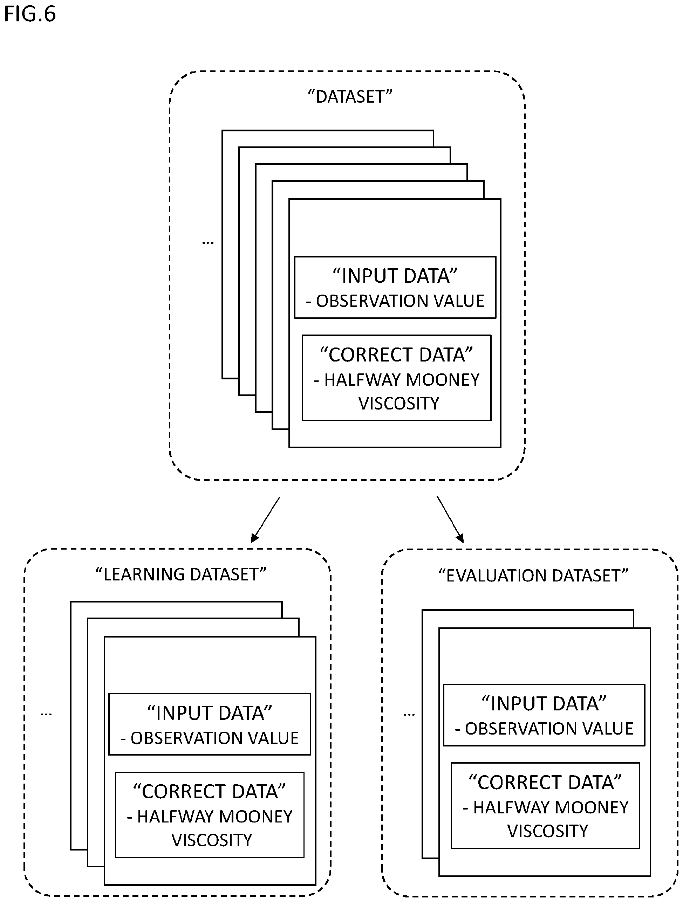

More specifically, in the machine learning, when the trained model is created, a "dataset" is used that is formed of a plurality of pairs of "input data", which is an observation value acquired in past manufacturing of the elastomer, and "correct data", which is a halfway Mooney viscosity of the elastomer acquired in the corresponding manufacturing, as shown in Figure 6. At the time, part of the "dataset" acquired through the past operation is used for a learning dataset for "learning", and the rest is used for an evaluation dataset for "evaluation" to check the learning. The learning dataset is also referred to as a training dataset. The evaluation dataset is also referred to as, for example, a test dataset.

Note that when the "dataset" is divided into the "learning dataset" and the "evaluation dataset", it is preferable to use chronologically older data for the "learning dataset" and newer data for the "evaluation dataset". For example, observation values acquired in manufacturing by the manufacturing device 1A can vary over time. For example, regarding observation values affected by aging of equipment, a plurality of observation values acquired at close times may be more similar to each other, than to an observation value acquired at a distant time. Accordingly, newer data tends to be similar to data to be acquired in future manufacturing. In such a case, by using a newer observation value in evaluation, it is more likely that the prediction model can be evaluated with the observation value that is more similar to an observation value in actual future manufacturing by the manufacturing device 1A. Accordingly, of the data acquired in the past manufacturing, for example, a predetermined proportion of older data is used for the learning dataset, and the rest newer data is used for the evaluation dataset, whereby the Mooney viscosity prediction model M1 can be evaluated with the data that is similar to data to be acquired in future manufacturing.

As shown in Figure 7A, when the target timing calculation model M2 is created, a "halfway Mooney viscosity" and a "target timing" that are actually measured are used for input data, and an actually measured "final Mooney viscosity" is used for correct data. Thus, the learner learns a relation among such halfway Mooney viscosities, target timings, and final Mooney viscosities, which is a learning dataset relation, and creates the target timing calculation model M2 that is a trained model indicating the learning dataset relation with parameters. As described above, a final Mooney viscosity is a Mooney viscosity actually measured for the elastomer acquired in past manufacturing. Accordingly, by using the learning dataset shown in Figure 7A, a relation about what performance the manufactured elastomer has in a case of each halfway Mooney viscosity and each target timing can be learned. As shown in Figure 7B, the thus created target timing calculation model M2 receives a "halfway Mooney viscosity" and a "target value" as inputs, and can obtain a "target timing" at which the final Mooney viscosity becomes the target value. The "halfway Mooney viscosity" as an input of the target timing calculation model M2 may be an actually measured value, or a highly accurate prediction value. Accordingly, a halfway Mooney viscosity obtained by the Mooney viscosity prediction model M1 may be used for an input of the target timing calculation model M2, or an input may be an actually measured halfway Mooney viscosity.

As for data used to create the target timing calculation model M2, a description of which using a drawing is omitted, it is also preferable to select a learning dataset from older data, and to select a test dataset from newer data, as described by using Figure 6.

Machine learning includes supervised learning, unsupervised learning, reinforcement learning, and the like, and the examples described with Figures 5A and 7A are supervised learning. The learner can use, for example, the above-described multiple regression analysis or the like for the machine learning. The learner may use another regression than the multiple regression analysis (for example, PCR (Principal Component Regression), PLS (Partial Least Squares), SVR (Support Vector Regression), DT (Decision Tree), RF (Random Forest), GPR (Gaussian Process Regression), NN (Neural Network), Model Tree, or the like). TD (Time Difference), JIT (Just In Time), or MW (Moving Window) may be prefixed to each of the above-mentioned algorithms. The learner may use LWPLS (Locally Weighted Partial Least Squares). The learner may use any of the above-mentioned schemes in combination.

[Embodiment 2]

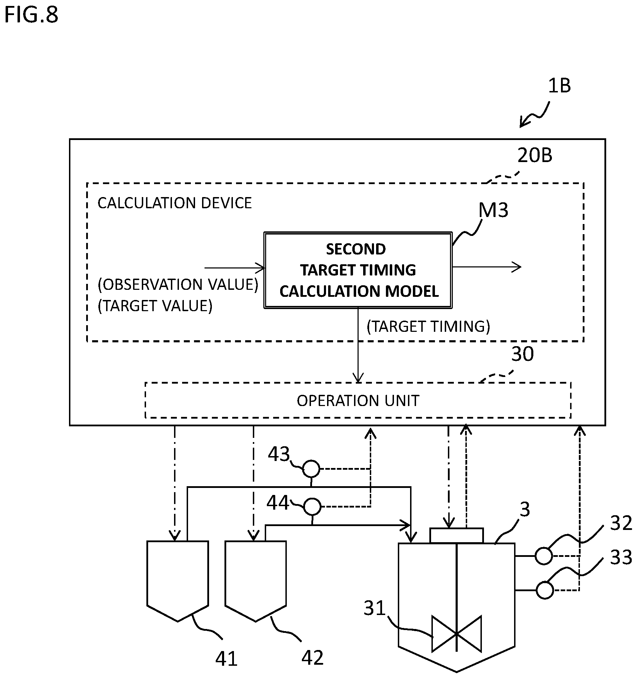

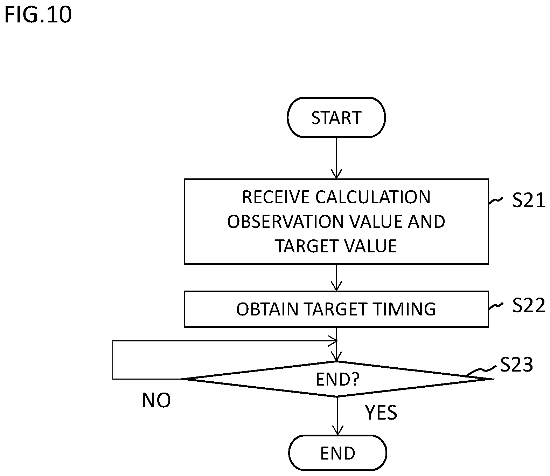

A manufacturing device 1B according to an embodiment 2 is described by using Figures 8 to 11B. Figure 8 is an example of the manufacturing device 1B used in manufacturing of an elastomer. The manufacturing device 1B shown in Figure 8 includes a calculation device 20B that obtains, in manufacturing of an elastomer that is manufactured by feeding raw materials into a polymerization tank 3, a target timing in the manufacturing of the elastomer by using a soft sensor. The manufacturing device 1B includes an operation unit 30 that performs a specific operation for terminating the current manufacturing, by using the target timing obtained by the calculation device 20B. Note that in the manufacturing device 1B according to the embodiment 2, a description of the same components as those of the manufacturing device 1A described above is omitted by using the same drawings. In the description of the manufacturing device 1B, it is also assumed that a raw material is fed intermittently or fed continuously.

Specifically, as shown in Figure 8, the calculation device 20B obtains a "target timing" in manufacturing of the elastomer from an "observation value", by using a second target timing calculation model M3 that is a third soft sensor. The manufacturing device 1B can finish feeding the material into the polymerization tank 3 by using the obtained target timing, and thus can terminate the manufacturing of the elastomer. Specifically, after the target timing is reached, the manufacturing device 1B can put the elastomer into an extractable state, by finishing stirring or finishing adjusting temperature in the polymerization tank 3.

<Calculation device>

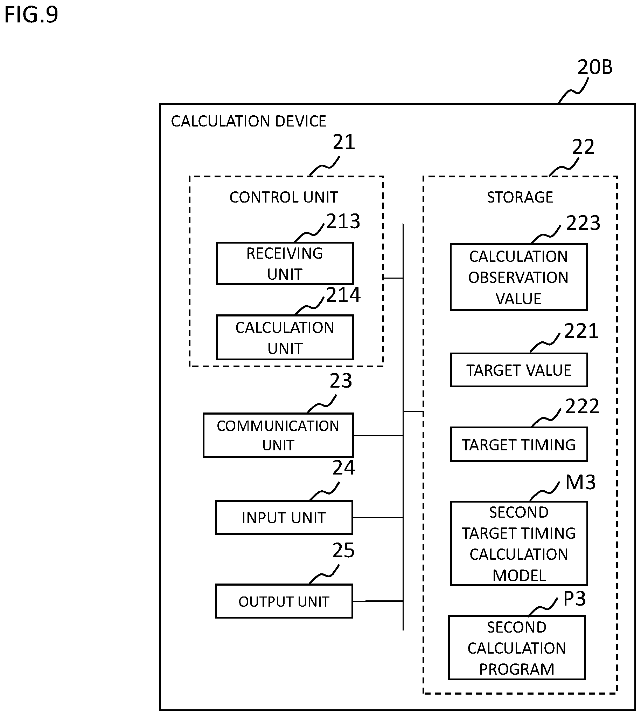

As shown in Figure 9, the calculation device 20B is an information processing device including a control unit 21, a storage 22, and a communication unit 23. Moreover, the calculation device 20B can include an input unit 24 and an output unit 25. For example, the control unit 21 implements processing as a receiving unit 213 and a calculation unit 214 by reading and executing a second calculation program P3 stored in the storage 22. The storage 22 stores, for example, a calculation observation value 223, a target value 221, a target timing 222, the second target timing calculation model M3, and the second calculation program P3. Note that the second target timing calculation model M3 may be included in the second calculation program P3.

The receiving unit 213 receives, as the calculation observation value 223, an observation value observed as a value related to manufacturing of the elastomer in current manufacturing. Note that the calculation observation value 223 may be identical to the prediction observation value 121 described above. The receiving unit 213 receives the target value 221 in the current manufacturing. The receiving unit 213 stores the received calculation observation value 223 and the received target value 221 in the storage 22.

The calculation unit 214 obtains a target timing in the current manufacturing, from the calculation observation value 223 and the target value 221 received by the receiving unit 213, by using a relation among an observation value acquired in past manufacturing of the same type of elastomer as the elastomer being manufactured, a final Mooney viscosity of the elastomer manufactured in the past manufacturing, and a target timing in the past manufacturing. Note that all of the observation value, the final Mooney viscosity, and the target timing in the past manufacturing are actually measured values.

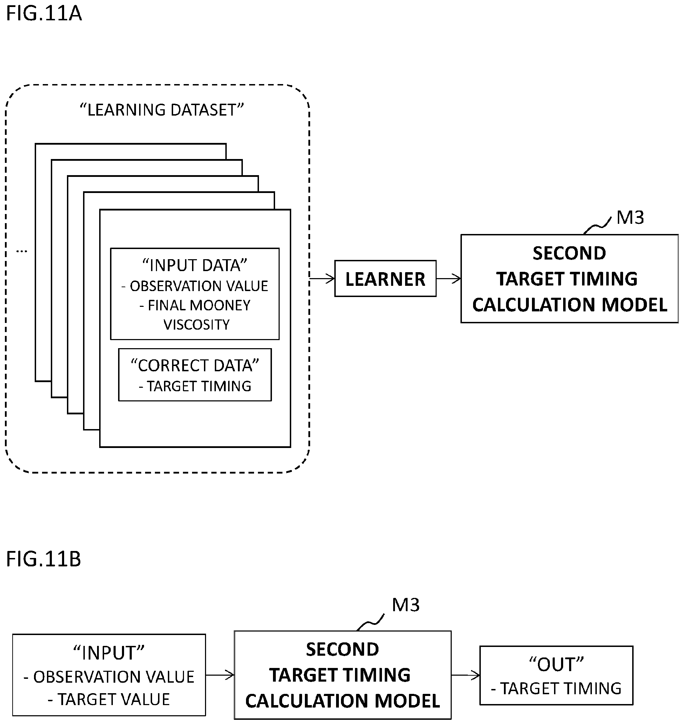

Here, in calculation of the target timing, the calculation unit 214 can use the second target timing calculation model M3, which is a trained model that has been trained through machine learning. The second target timing calculation model M3 has learned, through machine learning, a relation among a training observation value that is an observation value acquired in past manufacturing of the same type of elastomer as the elastomer being manufactured, a final Mooney viscosity of the elastomer acquired in the past manufacturing, and a target timing in the past manufacturing. Accordingly, the second target timing calculation model M3 can obtain the target timing used to determine termination of the current manufacturing of the elastomer, by using the calculation observation value and the target value received by the receiving unit 213, in order to allow performance of the elastomer being currently manufactured to be the target value. The machine learning will be described later by using Figure 11A.

Note that the calculation device 20B and the operation unit 30 are shown as separate entities in the example in Figure 8, but may be configured by using a single information processing device.

The target timing 222 is thus obtained by the calculation device 20B, whereby manufacturing can be terminated when the Mooney viscosity of the elastomer is in a target state. Accordingly, by using the target timing 222 obtained as a result of the calculation, the number of process steps related to actual measurement can be reduced. Note that even if the manufacturing is terminated by using the target timing obtained by the calculation device 20B by using the target value, the Mooney viscosity (final Mooney viscosity) of the acquired elastomer does not necessarily coincide with the target value, but comes within a desired range from the target value. By using the second target timing calculation model M3, variation in elastomer quality can be reduced, compared to a conventional simple soft sensor.

<Manufacturing processing>

Elastomer manufacturing processing in the manufacturing device 1B is described by using a flowchart of Figure 9. When the manufacturing device 1B starts manufacturing an elastomer, the receiving unit 213 of the calculation device 20B acquires a calculation observation value 223 and a target value 221 (S21). Note that the acquisition unit 111 may obtain the calculation observation value 223 through calculation by using a value received from an external sensor or device.

The calculation unit 214 obtains a target timing 222 used to determine termination of the manufacturing of the elastomer being manufactured, by using the calculation observation value 223 and the target value 221 received in step S21 (S22).

Thus, by using the calculation observation value 223 and the target timing 222 obtained in step S22, the manufacturing device 1B continues manufacturing of the elastomer until a condition for termination is met, and terminates the manufacturing of the elastomer when the condition is met (S5).

Thus, the manufacturing device 1B can reduce the number of process steps related to actual measurement, without needing a sample inspection, and can reduce variation in elastomer quality through an easy procedure.

<Machine learning>

An outline of creation and use of the second target timing calculation model M3 using machine learning is illustrated by using Figures 11A and 11B. As shown in Figure 11A, when the second target timing calculation model M3 is created, an "observation value" acquired in past manufacturing of the elastomer and a "final Mooney viscosity" actually measured for the elastomer acquired in the past manufacturing are used for input data, and a "target timing" actually measured in the past manufacturing of the elastomer is used for correct data. A learner learns a relation among such observation values, final Mooney viscosities, and target timings, which is a learning dataset relation, and creates the second target timing calculation model M3 that is a trained model indicating the learning dataset relation with parameters. As described above, a final Mooney viscosity is a Mooney viscosity actually measured for the elastomer acquired in past manufacturing. Accordingly, by using the learning dataset shown in Figure 11A, a relation about what target timing is set in a case of each observation value and each final Mooney viscosity can be indicated. As shown in Figure 11B, the thus created second target timing calculation model M3 receives an "observation value" and a "target value" as inputs, and can obtain a "target timing" at which the final Mooney viscosity becomes the target value.

<Advantageous effects and supplements>

As described above, each embodiment is described as illustration of the technique disclosed in the present application. However, the technique of the present disclosure is not limited to the described embodiments, and can be applied to embodiments in which a change, a substitution, an addition, an omission, or the like is made as appropriate.

A prediction device, a calculation device, a manufacturing device, and a manufacturing method according to all claims of the present disclosure are implemented by cooperation or the like with hardware resources, such as a processor and a memory, and a program.

The present application claims priority based upon Japanese Patent Application No. 2020-115514, filed on July 3, 2020 in Japan, the disclosure of which is incorporated herein in its entirety by reference.

Industrial Applicability

The prediction device, the calculation device, the manufacturing device, and the manufacturing method of the present disclosure are useful for, for example, manufacturing of a polymer.

Reference Signs List

1A, 1B Manufacturing device
10A Prediction device
20A, 20B Calculation device
11, 21 Control unit
12, 22 Storage
13, 23 Communication unit
14, 24 Input device
15, 25 Output device
111 Acquisition unit
112 Prediction unit
211, 213 Receiving unit
212, 214 Calculation unit
121 Prediction observation value
122 Halfway Mooney viscosity
221 Target value
222 Target timing
223 Calculation observation value

**Claims**

1. A prediction device that, in manufacturing of a polymer, predicts a performance value indicating performance of the polymer in a polymerization tank at a predetermined timing after a raw material is fed and before the manufacturing is terminated, comprising:

an acquisition unit that acquires, as a prediction observation value, an observation value observed, in current manufacturing of the polymer, as a value related to the manufacturing of the polymer; and

a prediction unit that predicts the performance value of the polymer being currently manufactured at the predetermined timing, from the prediction observation value acquired by the acquisition unit, by using a relation between an observation value acquired in past manufacturing of the same type of polymer as the polymer, and a performance value of the polymer at the predetermined timing in the past manufacturing.

2. The prediction device according to claim 1, wherein the prediction unit predicts the performance value of the polymer being currently manufactured at the predetermined timing, from the prediction observation value acquired by the acquisition unit, by using a trained model that has learned, through machine learning, a relation between a training observation value acquired in past manufacturing of the same type of polymer as the polymer, and a performance value of the polymer at the predetermined timing in the past manufacturing.

3. The prediction device according to claim 2, wherein

the acquisition unit acquires the observation value along with elapsed time since start of the current manufacturing, and uses a set of the elapsed time and the observation value for the prediction observation value, and

the prediction unit predicts the performance value of the polymer being currently manufactured at the predetermined timing, by using a relation between a training observation value that is a set of the observation value in the past manufacturing and elapsed time since start of the past manufacturing, and the performance value at the predetermined timing in the past manufacturing.

4. The prediction device-according to claim 2 or 3, wherein the acquisition unit predicts the performance value of the polymer at the predetermined timing, by using the trained model that is trained by using at least one observation value or performance value suitable for prediction of the performance value, the at least one observation value or performance value being selected, based on a genetic algorithm, from a plurality of types of observation values acquired in the past manufacturing and performance values of the polymer acquired in the past manufacturing.

5. The prediction device according to any one of claims 1 to 4, wherein the observation value includes at least any of a stirring electric current value of a stirrer that stirs a reaction mixture including the raw material in the polymerization tank, a stirring electric power value, a pressure value in the polymerization tank, an amount of any of raw materials fed from start of manufacturing until a current time point, and an amount of all raw materials fed from start of manufacturing until a current time point.

6. The prediction device according to any one of claims 1 to 5, wherein the performance value is any of a Mooney viscosity, a molecular weight, a specific gravity, a hardness, an elongation, a minimum torque, a maximum torque, an induction time to start of curing, an optimal curing time, a tensile strength, a 100% tensile stress, a glass-transition temperature, an initial pyrolysis temperature, and a compression set rate.

7. The prediction device according to any one of claims 1 to 6, wherein the polymer is an elastomer.

8. The prediction device according to any one of claims 1 to 7, wherein the predetermined timing is a timing at which a fed amount of a predetermined raw material of the polymer being manufactured becomes a predetermined value, or a timing at which a predetermined time period passes after start of manufacturing.

9. A calculation device that, in manufacturing of a polymer, obtains a target timing at which a specific operation is performed for terminating the manufacturing of the polymer in a polymerization tank, comprising:

a receiving unit that receives a target performance value indicating a target of performance of the polymer in current manufacturing, and a performance value at a predetermined timing after a raw material of the polymer is fed and before the manufacturing is terminated; and

a calculation unit that obtains the target timing used to determine termination of the manufacturing of the polymer, by using a relation among a performance value of the polymer at the predetermined timing in past manufacturing of the same type of polymer as the polymer, a performance value of the polymer acquired in the past manufacturing, and a target timing in the past manufacturing, and by using the target performance value and the performance value at the predetermined timing received by the receiving unit.

10. The calculation device according to claim 9, wherein the calculation unit obtains the target timing at which the polymer being currently manufactured achieves the target performance value, by using a second trained model that has learned, through machine learning, the relation among the performance value of the polymer at the predetermined timing in the past manufacturing of the same type of polymer as the polymer, the performance value of the polymer acquired in the past manufacturing, and the target timing in the past manufacturing, and by using the target performance value and the performance value at the predetermined timing.

11. The calculation device according to claim 9 or 10, wherein

the calculation device is connected to the prediction device according to claim 1 or 2, and the receiving unit receives, as the performance value at the predetermined timing, the performance value predicted by the prediction device.

12. A calculation device that, in manufacturing of a polymer, obtains a target timing used to determine termination of the manufacturing of the polymer, comprising:

a receiving unit that receives, as a calculation observation value, an observation value observed, in current manufacturing, as a value related to the manufacturing of the polymer, and receives, as a target value, a target performance value indicating a target of performance of the polymer in the current manufacturing; and a calculation unit that obtains a target timing in the current manufacturing, from the calculation observation value received by the receiving unit, by using a relation among an observation value acquired in past manufacturing of the same type of polymer as the polymer, a performance value of the polymer acquired in the past manufacturing, and a target timing in the past manufacturing.

13. The calculation device according to claim 12, wherein the calculation unit obtains the target timing, from the calculation observation value, by using a third trained model that has learned, through machine learning, a relation among a training observation value acquired in past manufacturing of the same type of polymer as the polymer, a performance value of the polymer acquired in the past manufacturing, and a target timing in the past manufacturing.

14. The calculation device according to any one of claims 9 to 13, wherein the polymer is an elastomer.

15. A manufacturing device comprising:

a polymerization tank into which a raw material of a polymer is fed; an acquisition unit that acquires, as a prediction observation value, an observation value observed, in current manufacturing of the polymer, as a value related to the manufacturing of the polymer; a prediction unit that predicts a performance value of the polymer being currently manufactured at a predetermined timing, from the prediction observation value acquired by the acquisition unit, by using a relation between an observation value acquired in past manufacturing of the same type of polymer as the polymer, and a performance value of the polymer at the predetermined timing in the past manufacturing; a receiving unit that receives a target performance value indicating a target of performance of the polymer in the current manufacturing, and the performance value at the predetermined timing predicted by the prediction unit; a calculation unit that obtains a target timing used to determine termination of the manufacturing of the polymer, by using a relation among the performance value of the polymer at the predetermined timing in the past manufacturing of the same type of polymer as the polymer, a performance value of the polymer acquired in the past manufacturing, and a target timing in the past manufacturing, and by using the target performance value and the performance value at the predetermined timing received by the receiving unit; and an operation unit that performs a specific operation for terminating the current manufacturing, by using the target timing obtained by the calculation unit.

16. A method for manufacturing a polymer, comprising:

feeding a raw material of a polymer into a polymerization tank; acquiring, as a prediction observation value, an observation value observed, in current manufacturing of the polymer, as a value related to the manufacturing of the polymer; predicting a performance value of the polymer being currently manufactured at a predetermined timing, from the acquired prediction observation value, by using a relation between an observation value acquired in past manufacturing of the same type of polymer as the polymer, and a performance value of the polymer at the predetermined timing in the past manufacturing; receiving a target performance value indicating a target of performance of the polymer in the current manufacturing, and the predicted performance value at the predetermined timing; obtaining a target timing used to determine termination of the manufacturing of the polymer, by using a relation among the performance value of the polymer at the predetermined timing in the past manufacturing of the same type of polymer as the polymer, a performance value of the polymer acquired in the past manufacturing, and a target timing in the past manufacturing, and by using the received target performance value and the received performance value at the predetermined timing; and
terminating the current manufacturing, by using the obtained target timing.

**FIG.1**

# FIG.2

10A

**PREDICTION DEVICE**

11 — **CONTROL UNIT**

111 — ACQUISITION UNIT

112 — STORAGE UNIT

13 — COMMUNICATION UNIT

14 — INPUT UNIT

15 — OUTPUT UNIT

12 — **STORAGE**

121 — PREDICTION OBSERVATION VALUE

122 — HALFWAY MOONEY VISCOSITY

M1 — MOONEY VISCOSITY PREDICTION MODEL

P1 — PREDICTION PROGRAM

FIG.3

CALCULATION DEVICE — 20A

CONTROL UNIT — 21
- RECEIVING UNIT — 211
- CALCULATION UNIT — 212

COMMUNICATION UNIT — 23

INPUT UNIT — 24

OUTPUT UNIT — 25

STORAGE — 22
- HALFWAY MOONEY VISCOSITY — 122
- TARGET VALUE — 221
- TARGET TIMING — 222
- TARGET TIMING CALCULATION MODEL — M2
- CALCULATION PROGRAM — P2

FIG.4

```
                    ┌─────────────┐
                    │    START    │
                    └─────────────┘
                           │
   ┌ ─ ─ ─ ─ ─ ─ ─ ─ ─ ─ ─│─ ─ ─ ─ ─ ─ ─ ─ ─ ─ ─┐
     PREDICTION           │
   │                      ▼                       │
        ┌───────────────────────────────┐
   │    │     ACQUIRE PREDICTION         │  ⌇ S11 │
        │     OBSERVATION VALUE          │
   │    └───────────────────────────────┘        │
                          │
   │                      ▼                       │
        ┌───────────────────────────────┐
   │    │    OBTAIN HALFWAY MOONEY       │  ⌇ S12 │
        │    VISCOSITY                   │
   │    └───────────────────────────────┘        │
   └ ─ ─ ─ ─ ─ ─ ─ ─ ─ ─ ─│─ ─ ─ ─ ─ ─ ─ ─ ─ ─ ─┘
   ┌ ─ ─ ─ ─ ─ ─ ─ ─ ─ ─ ─│─ ─ ─ ─ ─ ─ ─ ─ ─ ─ ─┐
     CALCULATION          │
   │                      ▼                       │
        ┌───────────────────────────────┐
   │    │   RECEIVE HALFWAY MOONEY       │  ⌇ S13 │
        │   VISCOSITY AND TARGET VALUE   │
   │    └───────────────────────────────┘        │
                          │
   │                      ▼                       │
        ┌───────────────────────────────┐
   │    │    OBTAIN TARGET TIMING        │  ⌇ S14 │
        └───────────────────────────────┘
   └ ─ ─ ─ ─ ─ ─ ─ ─ ─ ─ ─│─ ─ ─ ─ ─ ─ ─ ─ ─ ─ ─┘
   ┌────────────────────►  │
   │                      ▼
   │               ◇───────────────◇   ⌇ S15
   └───────────────     END?          
        NO         ◇───────────────◇
                          │   YES
                          ▼
                    ┌─────────────┐
                    │     END     │
                    └─────────────┘
```

FIG.5A

"LEARNING DATASET"

"INPUT DATA"
- OBSERVATION VALUE

"CORRECT DATA"
- HALFWAY MOONEY
VISCOSITY

...

LEARNER

M1

**MOONEY VISCOSITY
PREDICTION MODEL**

FIG.5B

"INPUT"
- OBSERVATION VALUE

M1

**MOONEY VISCOSITY
PREDICTION MODEL**

"OUTPUT"
- HALFWAY MOONEY
VISCOSITY

FIG.6

"DATASET"

...

"INPUT DATA"
- OBSERVATION VALUE

"CORRECT DATA"
- HALFWAY MOONEY
VISCOSITY

"LEARNING DATASET"

...

"INPUT DATA"
- OBSERVATION VALUE

"CORRECT DATA"
- HALFWAY MOONEY
VISCOSITY

"EVALUATION DATASET"

...

"INPUT DATA"
- OBSERVATION VALUE

"CORRECT DATA"
- HALFWAY MOONEY
VISCOSITY

FIG.7A

```
┌ ─ ─ ─ ─ ─ ─ ─ ─ ─ ─ ─ ─ ─ ─ ─ ─ ┐
          "LEARNING DATASET"
```

"INPUT DATA"
- HALFWAY
MOONEY VISCOSITY
- TARGET TIMING

"CORRECT DATA"
- FINAL
MOONEY VISCOSITY

LEARNER

M2
TARGET TIMING
CALCULATION MODEL

FIG.7B

"INPUT"
- HALFWAY
MOONEY VISCOSITY
- TARGET VALUE

M2
TARGET TIMING
CALCULATION MODEL

"OUTPUT"
- TARGET TIMING

FIG.8

1B

20B

CALCULATION DEVICE

M3

(OBSERVATION VALUE)
(TARGET VALUE)

SECOND
TARGET TIMING
CALCULATION MODEL

(TARGET TIMING)

30

OPERATION UNIT

43

44

3

32
33

31

41    42

FIG.9

FIG.10

FIG.11A

"LEARNING DATASET"

"INPUT DATA"
- OBSERVATION VALUE
- FINAL MOONEY VISCOSITY

"CORRECT DATA"
- TARGET TIMING

LEARNER

M3

SECOND TARGET TIMING CALCULATION MODEL

FIG.11B

"INPUT"
- OBSERVATION VALUE
- TARGET VALUE

M3

SECOND TARGET TIMING CALCULATION MODEL

"OUT"
- TARGET TIMING

<table>
<tr><td colspan="2" align="center">**INTERNATIONAL SEARCH REPORT**</td><td>International application No.</td></tr>
<tr><td colspan="2"></td><td>PCT/JP2021/025156</td></tr>
</table>

A.   CLASSIFICATION OF SUBJECT MATTER
C08F 14/18(2006.01)i; C08F 2/01(2006.01)i; G01N 33/44(2006.01)i
FI: C08F2/01; C08F14/18; G01N33/44
According to International Patent Classification (IPC) or to both national classification and IPC

B.   FIELDS SEARCHED

Minimum documentation searched (classification system followed by classification symbols)
C08F; G01N33/44

Documentation searched other than minimum documentation to the extent that such documents are included in the fields searched
    Published examined utility model applications of Japan          1922–1996
    Published unexamined utility model applications of Japan        1971–2021
    Registered utility model specifications of Japan                1996–2021
    Published registered utility model applications of Japan        1994–2021

Electronic data base consulted during the international search (name of data base and, where practicable, search terms used)

C.   DOCUMENTS CONSIDERED TO BE RELEVANT

| Category* | Citation of document, with indication, where appropriate, of the relevant passages | Relevant to claim No. |
|---|---|---|
| X<br>Y | JP 2007-191657 A (HITACHI, LTD.) 02 August 2007 (2007-08-02) claims, paragraphs [0016]-[0018] | 1, 5, 6, 8<br>2, 3, 4 |
| X<br>Y | JP 5-140230 A (MITSUBISHI KASEI CORP.) 08 June 1993 (1993-06-08) claims, paragraphs [0022], [0030]-[0034] | 1, 5, 6, 8<br>2-4 |
| X<br>Y | JP 2019-178256 A (MITSUI CHEMICALS, INC.) 17 October 2019 (2019-10-17) claims, paragraphs [0003], [0004], [0023], [0034], [0039], [0047] | 1, 5-8<br>2-4 |
| X<br>Y | JP 2011-525967 A (THE ADMINISTRATORS OF THE TULANE EDUCATIONAL FUND) 29 September 2011 (2011-09-29) claims, paragraph [0117], fig. 14 | 1, 5, 6, 8<br>2-4 |

☒   Further documents are listed in the continuation of Box C.          ☒   See patent family annex.

| | |
|---|---|
| *      Special categories of cited documents: | "T"   later document published after the international filing date or priority date and not in conflict with the application but cited to understand the principle or theory underlying the invention |
| "A"   document defining the general state of the art which is not considered to be of particular relevance | |
| "E"   earlier application or patent but published on or after the international filing date | "X"   document of particular relevance; the claimed invention cannot be considered novel or cannot be considered to involve an inventive step when the document is taken alone |
| "L"   document which may throw doubts on priority claim(s) or which is cited to establish the publication date of another citation or other special reason (as specified) | "Y"   document of particular relevance; the claimed invention cannot be considered to involve an inventive step when the document is combined with one or more other such documents, such combination being obvious to a person skilled in the art |
| "O"   document referring to an oral disclosure, use, exhibition or other means | |
| "P"   document published prior to the international filing date but later than the priority date claimed | "&"   document member of the same patent family |

| Date of the actual completion of the international search | Date of mailing of the international search report |
|---|---|
| 13 September 2021 (13.09.2021) | 21 September 2021 (21.09.2021) |

| Name and mailing address of the ISA/ | Authorized officer |
|---|---|
| Japan Patent Office<br>3-4-3, Kasumigaseki, Chiyoda-ku,<br>Tokyo 100-8915, Japan | |
| | Telephone No. |

Form PCT/ISA/210 (second sheet) (January 2015)

**INTERNATIONAL SEARCH REPORT**

| International application No. |
|---|
| PCT/JP2021/025156 |

C (Continuation).   DOCUMENTS CONSIDERED TO BE RELEVANT

| Category* | Citation of document, with indication, where appropriate, of the relevant passages | Relevant to claim No. |
|---|---|---|
| X<br>Y | JP 2003-76934 A (TOSOH CORP.) 14 March 2003 (2003-03-14) claims, paragraphs [0019]-[0023] | 1, 5-8<br>2-4 |
| Y | WO 2020/054183 A1 (FUJIFILM CORPORATION) 19 March 2020 (2020-03-19) claims, paragraphs [0002]-[0005] | 2, 3 |
| Y | JP 2015-520674 A (THE UNIVERSITY COURT OF THE UNIVERSITY OF GLASGOW) 23 July 2015 (2015-07-23) claims | 4 |
| A | JP 2018-162410 A (MITSUI CHEMICALS, INC.) 18 October 2018 (2018-10-18) entire text | 1-8 |
| A | JP 2001-294603 A (MITSUI CHEMICALS, INC.) 23 October 2001 (2001-10-23) entire text | 1-8 |
| A | JP 4-320405 A (SUMITOMO HEAVY INDUSTRIES, LTD.) 11 November 1992 (1992-11-11) entire text | 1-8 |

**INTERNATIONAL SEARCH REPORT**

| International application No. |
|---|
| PCT/JP2021/025156 |

**Box No. II**        **Observations where certain claims were found unsearchable (Continuation of item 2 of first sheet)**

This international search report has not been established in respect of certain claims under Article 17(2)(a) for the following reasons:

1. ☐ Claims Nos.:
because they relate to subject matter not required to be searched by this Authority, namely:

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**Box No. III**        **Observations where unity of invention is lacking (Continuation of item 3 of first sheet)**

This International Searching Authority found multiple inventions in this international application, as follows:
See extra sheet

1. ☐ As all required additional search fees were timely paid by the applicant, this international search report covers all searchable claims.

2. ☐ As all searchable claims could be searched without effort justifying additional fees, this Authority did not invite payment of additional fees.

3. ☐ As only some of the required additional search fees were timely paid by the applicant, this international search report covers only those claims for which fees were paid, specifically claims Nos.:

4. ☒ No required additional search fees were timely paid by the applicant.   Consequently, this international search report is restricted to the invention first mentioned in the claims; it is covered by claims Nos.: 1-8

**Remark on Protest**        ☐ The additional search fees were accompanied by the applicant's protest and, where applicable, the payment of a protest fee.

☐ The additional search fees were accompanied by the applicant's protest but the applicable protest fee was not paid within the time limit specified in the invitation.

☐ No protest accompanied the payment of additional search fees.

Form PCT/ISA/210 (continuation of first sheet (2)) (January 2015)

| | INTERNATIONAL SEARCH REPORT<br>Information on patent family members | | International application No.<br>PCT/JP2021/025156 |

| Patent Documents<br>referred in the<br>Report | Publication<br>Date | Patent Family | Publication<br>Date |
|---|---|---|---|
| JP 2007-191657 A | 02 Aug. 2007 | (Family: none) | |
| JP 5-140230 A | 08 Jun. 1993 | (Family: none) | |
| JP 2019-178256 A | 17 Oct. 2019 | (Family: none) | |
| JP 2011-525967 A | 29 Sep. 2011 | US 2009/0306311 A1<br>claims, paragraph<br>[0241], fig. 14<br>US 2016/0033470 A1<br>EP 2294406 A1<br>KR 10-2011-0053945 A | |
| JP 2003-76934 A | 14 Mar. 2003 | (Family: none) | |
| WO 2020/054183 A1 | 19 Mar. 2020 | (Family: none) | |
| JP 2015-520674 A | 23 Jul. 2015 | US 2015/0133306 A1<br>claims<br>WO 2013/175240 A1<br>EP 2855008 A1<br>CN 104507564 A | |
| JP 2018-162410 A | 18 Oct. 2018 | (Family: none) | |
| JP 2001-294603 A | 23 Oct. 2001 | (Family: none) | |
| JP 4-320405 A | 11 Nov. 1992 | (Family: none) | |

**INTERNATIONAL SEARCH REPORT**

| International application No. |
| --- |
| PCT/JP2021/025156 |

<Continuation of Box No. III>

Invention A: Claims 1-8
Invention B: Claims 9-11
Invention C: Claims 12-14
Invention D: Claims 15-16

(Invention A) Claims 1-8, which refer to claim 1, form an invention group, and are thus classified as invention 1.

(Invention B) Claims 9-11 share, with claim 1 classified as invention 1, the common technical feature of "using an observation value obtained during past preparation, in a device for use in polymer preparation".

However, this technical feature does not make a contribution over the prior art in light of the disclosure of documents 1-9, and is thus not considered to be a special technical feature. Furthermore, these inventions share no other identical or corresponding special technical features.

Therefore, claims 9-11 are classified as invention 2.

(Invention C) Claims 12-14 share, with claim 1 classified as invention 1, the common technical feature of "using an observation value obtained during past preparation, in a device for use in polymer preparation".

However, this technical feature does not make a contribution over the prior art in light of the disclosure of documents 1-9, and is thus not considered to be a special technical feature. Furthermore, these inventions share no other identical or corresponding special technical features.

Claims 12-14 share, with claim 9 classified as invention 2, the common technical feature of a "calculation device for obtaining target timing of finishing polymer preparation when preparing a polymer, the calculation device being provided with a calculation unit for obtaining the target timing by using the relationship between a performance value obtained during past preparation and the target timing of the past preparation".

However, this technical feature does not make a contribution over the prior art in light of the disclosure of documents 1 and 7-9, and is thus not considered to be a special technical feature. Furthermore, these inventions share no other identical or corresponding special technical features.

Therefore, claims 12-14 are classified as invention 3.

(Invention D) Claims 15-16 share, with claim 1 classified as invention 1, the common technical feature of "acquiring, as an observation value for prediction, an observation value observed as a value related to preparation of a polymer that is currently being prepared, and using the relationship between the observation value obtained during past preparation of a polymer of the same type as the above polymer and a performance value of the polymer at prescribed timing during the past preparation to predict the performance value, at the prescribed timing, of the polymer being currently prepared from the observation value for prediction acquired by the acquisition unit, with regard to polymer preparation".

However, this technical feature does not make a contribution over the prior art in light of the disclosure of documents 1-9, and is thus not considered to be a special technical feature. Furthermore, these inventions share no other identical or corresponding special technical features.

**INTERNATIONAL SEARCH REPORT**

International application No.

PCT/JP2021/025156

Claims 15-16 share, with claim 12 classified as invention 3, the common technical feature of a "device for obtaining target timing of finishing polymer preparation, the device being provided with: a reception unit, which receives a target performance value indicating the target of performance of a polymer during current preparation; and a calculation unit, which calculates using the target performance value received by the reception unit and the relationship between the performance value of a polymer obtained during past preparation and target timing of the past preparation".

However, this technical feature does not make a contribution over the prior art in light of the disclosure of documents 1 and 9, and is thus not considered to be a special technical feature. Furthermore, these inventions share no other identical or corresponding special technical features.

Claims 15-16 share, with claim 9 classified as invention 2, the common technical feature of a "device for obtaining target timing of finishing polymer preparation, the device being provided with: a reception unit, which receives a target performance value indicating the target of performance of a polymer during current preparation; and a calculation unit, which calculates the target timing used in determining to finish preparation of the polymer by using the target performance value received by the reception unit and the relationship between the performance value, at the prescribed timing, of a polymer of the same type as the above polymer during past preparation, the performance value of the polymer obtained during the past preparation, and target timing of the past preparation". Since these matters have a special technical feature, claims 15-16 are classified as invention 2.

Therefore, claims 1-16 of the present application are classified into the three inventions.
Invention 1: Claims 1-8
Invention 2: Claims 9-11 and claims 15-16
Invention 3: Claims 12-14

List of documents:
1. JP 2007-191657 A (HITACHI, LTD.) 02 August 2007 (2007-08-02)
2. JP 5-140230 A (MITSUBISHI KASEI CORP.) 08 June 1993 (1993-06-08)
3. JP 2019-178256 A (MITSUI CHEMICALS, INC.) 17 October 2019 (2019-10-17)
4. JP 2011-525967 A (THE ADMINISTRATORS OF THE TULANE EDUCATIONAL FUND) 29 September 2011 (2011-09-29)
5. JP 2003-76934 A (TOSOH CORP.) 14 March 2003 (2003-03-14)
6. WO 2020/054183 A1 (FUJIFILM CORPORATION) 19 March 2020 (2020-03-19)
7. JP 2018-162410 A (MITSUI CHEMICALS, INC.) 18 October 2018 (2018-10-18)
8. JP 2001-294603 A (MITSUI CHEMICALS, INC.) 23 October 2001 (2001-10-23)
9. JP 4-320405 A (SUMITOMO HEAVY INDUSTRIES, LTD.) 11 November 1992 (1992-11-11)

Form PCT/ISA/210 (extra sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- JP 2011245742 A
- JP 2020115514 A